# EUROPEAN PATENT APPLICATION

(11) **EP 4 450 097 A1**
(43) Date of publication of application: **23.10.2024**
(21) Application number: 22907779.7
(22) Date of filing: 01.12.2022
(51) Int. Cl.: A61M 5/142, A61M 5/172, A61B 5/145, A61B 5/157, A61B 5/00

(54) **LIQUID MEDICINE INJECTION APPARATUS**

(30) Priority: 17.12.2021 KR 20210181639
(71) Applicant: Eoflow Co., Ltd., Seongnam-si, Gyeonggi-do 13605 (KR)
(72) Inventor: KIM, Jesse Jaejin, Seongnam-si Gyeonggi-do 13562 (KR); HAN, Yong Joon, Seoul 05236 (KR)
(74) Representative: HGF
(86) International application number: PCT/KR2022/019326
(87) International publication number: WO 2023/113310

(57) **Abstract**

Provided is a Liquid medicine injection apparatus including a needle portion having one or more needle bodies configured to be injected into a user's body, a reservoir portion configured to accommodate a medical liquid and receive power from a driving portion to deliver the medical liquid to the needle portion, a base portion on which the reservoir portion, the needle portion, and the driving portion are installed, a cover portion covering the base portion, and a sensor portion configured to measure blood sugar, wherein, in the cover portion, an insertion groove which has one side open and has a preset depth is formed, and the sensor portion is insertable into the insertion groove and is spatially separable from the base portion.

## Description

### Technical Field

The present disclosure relates to a Liquid medicine injection apparatus.

### [Background Art]

In general, a Liquid medicine injection apparatus such as an insulin injection apparatus is used to inject a medical liquid into a patient's body. Such a Liquid medicine injection apparatus is used by medical professionals such as a doctor or a nurse, but is mostly used by an ordinary person such as a patient himself/herself or a caregiver.

In case of diabetic patients, particularly diabetic children, it is necessary to inject a medical liquid such as insulin into a human body at regular intervals. Thus, a patch-type Liquid medicine injection apparatus that is attached to a human body for a predetermined period of time to be used has been researched and developed. Such a Liquid medicine injection apparatus may be used as a patch type while being attached to the abdomen or waist of a patient for a predetermined period of time.

There is the need to control precise injection of a medical liquid from a Liquid medicine injection apparatus into the body of a user such as a patient, and it is important to precisely inject a small amount of medical liquid through a compact Liquid medicine injection apparatus.

In conventional Liquid medicine injection apparatuses, a sensor portion including a sensor needle that penetrates the skin of the user and a needle portion through which a medical liquid is injected are installed together on a single base portion, so even if the sensor portion does not need to be replaced, the sensor portion has to be removed from the skin of the user to replace a reservoir portion, a battery portion, etc.

In addition, the usage period of the sensor portion is relatively longer than the storage period of the medical liquid accommodated in the reservoir portion, and due to this difference in period, the reservoir portion had to be replaced, and the sensor portion is installed in the base portion together with the reservoir portion, and the needle body inserted into the skin of the user is also removed from the skin of the user.

Here, if the base portion on which the needle portion is arranged is coupled to the sensor portion while the position of the sensor portion is maintained, the needle body is inevitably inserted in the same position as the position where it was previously inserted into the skin of the user, and the skin tissue where the needle body was inserted is prone to clumping, and the insulin absorption rate is low until these agglomerations were resolved.

### Detailed Description of the Invention

### Technical Objective

The present disclosure provides a Liquid medicine injection apparatus in which a sensor portion is insertable into an insertion groove formed in a cover portion, and thus the sensor portion is spatially separable from a base portion, and the sensor portion and internal components such as a reservoir portion, a needle portion, a driving portion, and a battery portion may be independently installed on the skin of a user.

In addition, the present disclosure provides a Liquid medicine injection apparatus in which a base portion where internal components are installed and a sensor portion are provided independently of each other to thereby improve convenience of use and convenience of replacement.

The present disclosure also provides a Liquid medicine injection apparatus in which a base portion and a cover portion connected to the base portion may be coupled to a sensor portion in varying directions, so that the insertion position of a needle body on the skin of the user may be varied while maintaining the position of the sensor portion, and thus a decrease in the insulin absorption rate due to insertion of the needle body may be prevented.

### Technical Solution

According to an aspect of the present disclosure, there is provided a Liquid medicine injection apparatus including: a needle portion having one or more needle bodies configured to be injected into a user's body; a reservoir portion configured to accommodate a medical liquid and receive power from a driving portion to deliver the medical liquid to the needle portion; a base portion on which the reservoir portion, the needle portion, and the driving portion are installed; a cover portion covering the base portion; and a sensor portion configured to measure blood sugar, wherein, in the cover portion, an insertion groove which has one side open and has a preset depth is formed, and the sensor portion is insertable into the insertion groove and is spatially separable from the base portion.

In addition, the Liquid medicine injection apparatus may further include an attachment portion which is connected to the base portion and the sensor portion and attachable to the user.

Additionally, the attachment portion may include: a first attachment member connected to one side of the base portion; and a second attachment member that is formed as a separate component from the first attachment member and is connected to one side of the sensor portion.

In addition, the Liquid medicine injection apparatus may further include a lever portion rotatably installed on the cover portion and configured to transmit rotational power to the needle portion.

In addition, the sensor portion may include: a sensor frame that is insertable into the insertion groove; and a sensor module installed in the sensor frame and including a sensor needle.

In addition, the sensor module may include: a sensor body in which the sensor needle is formed; and a pin portion connected to the sensor body and including a conductive material; a sensor cover that covers the pin portion and is connected to the sensor frame.

In addition, the pin portion and the sensor cover may be each provided in plurality and disposed on both sides of the sensor body.

In addition, the Liquid medicine injection apparatus may further include a circuit portion electrically connected to the driving portion.

Additionally, the base portion may include: a first base connected to one side of the cover portion and on which the circuit portion is disposed; and a second base disposed on the first base and configured to fix positions of the reservoir portion, the needle portion, and the driving portion.

Additionally, the circuit portion may include: a first circuit disposed on the first base; and a second circuit connected to the first circuit and disposed on an outer peripheral surface of the second base extending from the first base toward the cover portion.

### [Effects of the Invention]

In a Liquid medicine injection apparatus according to an embodiment of the present disclosure, a sensor portion may be provided independently of and spatially separated from a base portion, and a circuit portion and the sensor portion may be electrically connected to each other by inserting the sensor portion into an insertion groove formed in the cover portion.

In addition, as the sensor portion is spatially separated from the base portion, the sensor portion may be first installed separately on the skin of a user, and then the base portion, where internal components such as a reservoir portion, a needle portion, and a driving portion are installed, may be installed on the skin of the user.

In addition, when it is necessary to replenish a medical liquid or replace a battery portion or the needle portion, the base portion may be independently separated from the skin of the user and replaced while the sensor portion is maintained in a state of being installed on the skin of the user, and thus, convenience of use may be improved.

In addition, by coupling, to the sensor portion, the base portion on which the needle portion is arranged and the cover portion connected to the base portion, in varying directions, while maintaining the position of the sensor portion, the insertion position of a needle body on the skin of the user may be changed and a decrease in an insulin absorption rate due to insertion of the needle body at the same position may be prevented.

However, the scope of the present disclosure is not limited by the above-described effects.

### [Brief Description of the Drawings]

FIG. 1 is a perspective view illustrating a Liquid medicine injection apparatus according to an embodiment of the present disclosure.
FIG. 2 is a diagram illustrating a separated state of the Liquid medicine injection apparatus according to an embodiment of the present disclosure.
FIG. 3 is a bottom view illustrating a base portion to which an attachment portion is connected, according to an embodiment of the present disclosure.
FIG. 4 is a diagram illustrating portion A of FIG. 3.
FIG. 5 is a view illustrating a side view of the base portion according to an embodiment of the present disclosure.
FIG. 6 is a bottom perspective view illustrating a Liquid medicine injection apparatus according to an embodiment of the present disclosure, from which a first base is removed.
FIG. 7 is an exploded view illustrating a sensor portion according to an embodiment of the present disclosure.
FIG. 8 is a cross-sectional view taken along line I-I of FIG. 1.
FIG. 9 is a diagram illustrating a sensor portion according to an embodiment of the present disclosure.
FIG. 10 is a diagram illustrating a path through which a cover portion covers the sensor portion, according to an embodiment of the present disclosure.

### [Best Mode for Carrying out the Invention]

As the present disclosure allows for various changes and numerous embodiments, particular embodiments will be illustrated in the drawings and described in detail in the written description. The effects and features of the present disclosure, and ways to achieve them will become apparent by referring to embodiments that will be described later in detail with reference to the drawings. However, the present disclosure is not limited to the following embodiments but may be embodied in various forms.

Hereinafter, embodiments of the present disclosure will be described in detail with reference to the accompanying drawings, and in the description with reference to the drawings, like reference numerals refer to like elements and redundant descriptions thereof will be omitted.

Singular expressions, unless defined otherwise in contexts, include plural expressions.

In the embodiments below, it will be further understood that the terms "comprise" and/or "have" used herein specify the presence of stated features or elements, but do not preclude the presence or addition of one or more other features or elements.

When an embodiment is implementable in another manner, a predetermined process order may be different from a described one. For example, two processes that are consecutively described may be substantially simultaneously performed or may be performed in an opposite order to the described order.

Also, in the drawings, for convenience of description, sizes of elements may be exaggerated or contracted. For example, since sizes and thicknesses of elements in the drawings are arbitrarily illustrated for convenience of explanation, the following embodiments are not limited thereto.

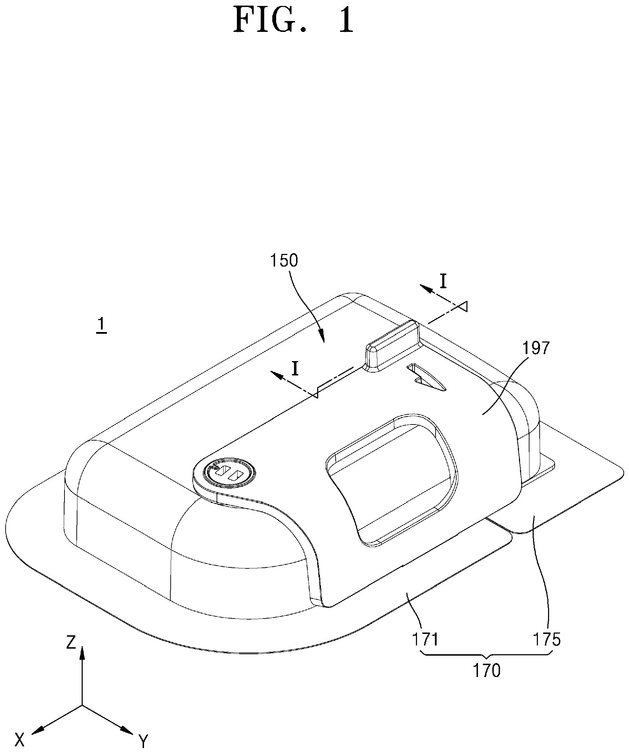

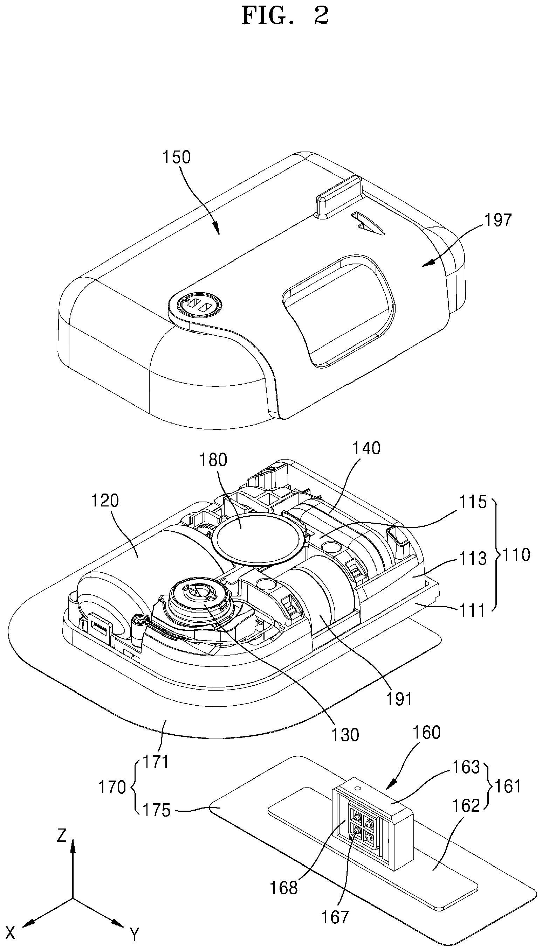

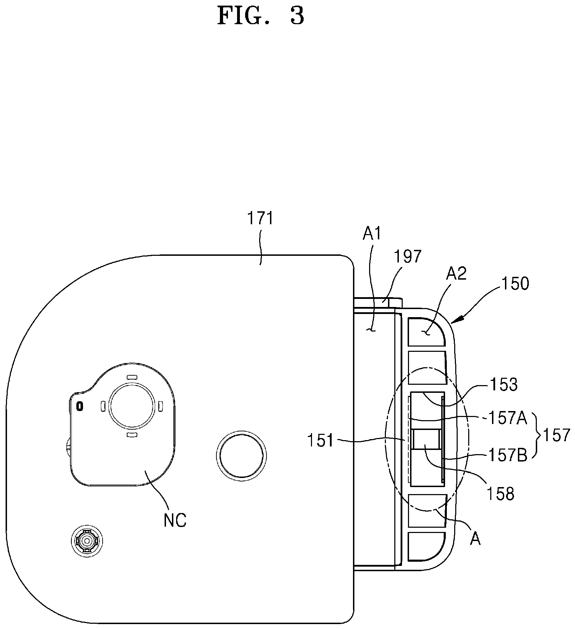

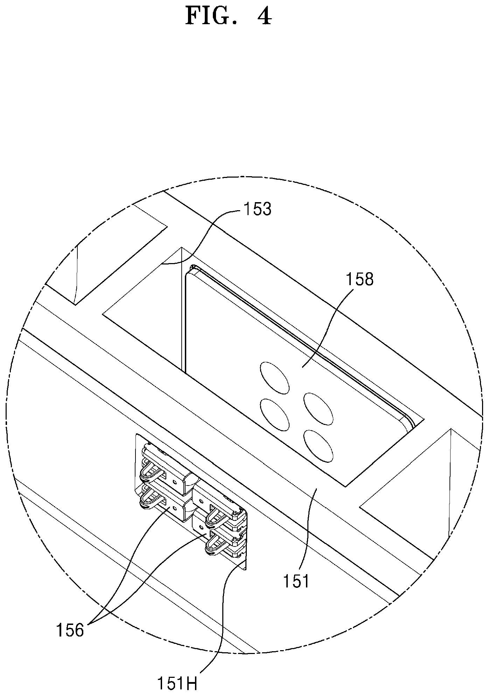

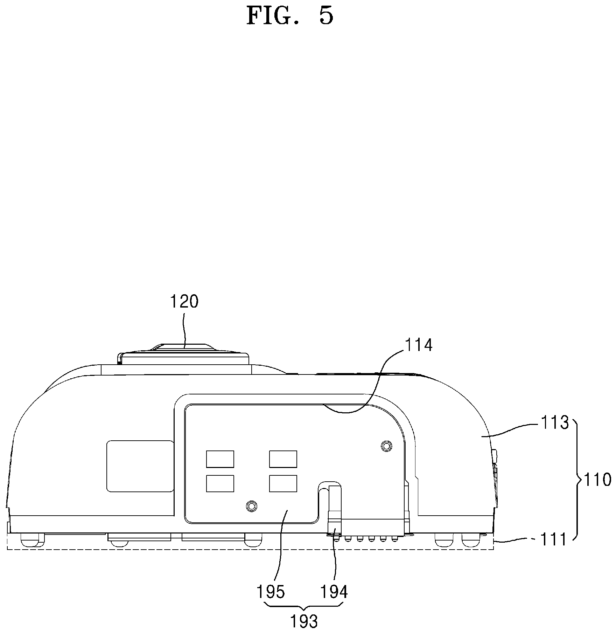

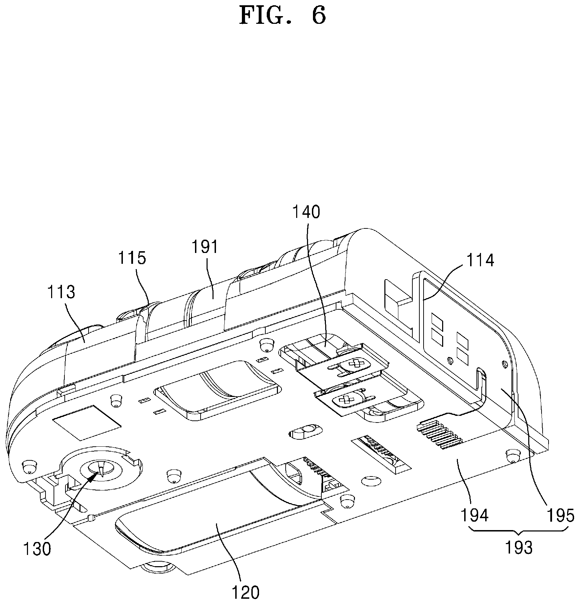

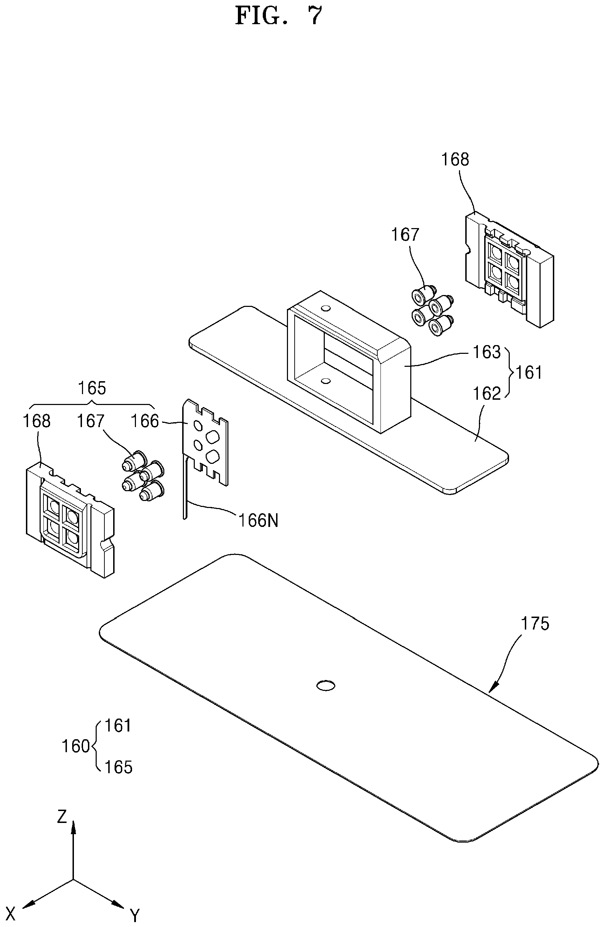

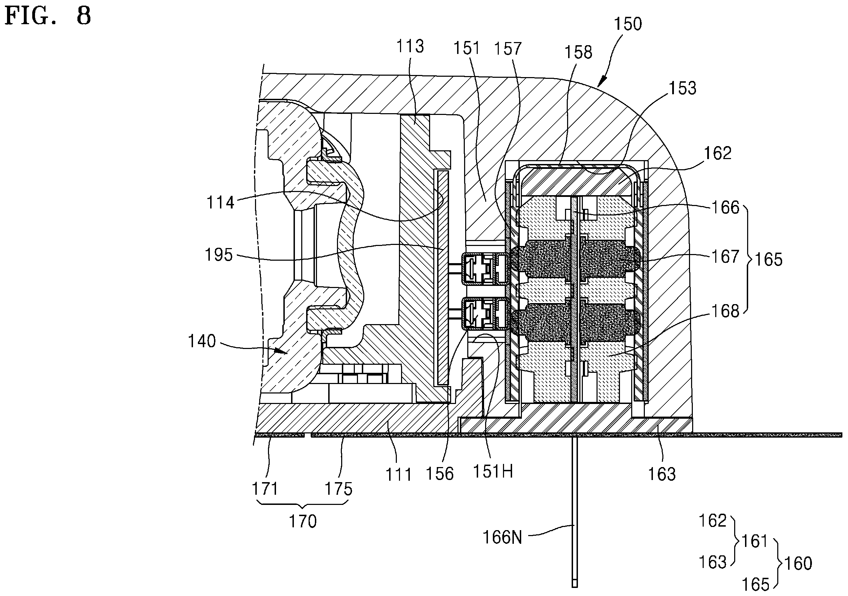

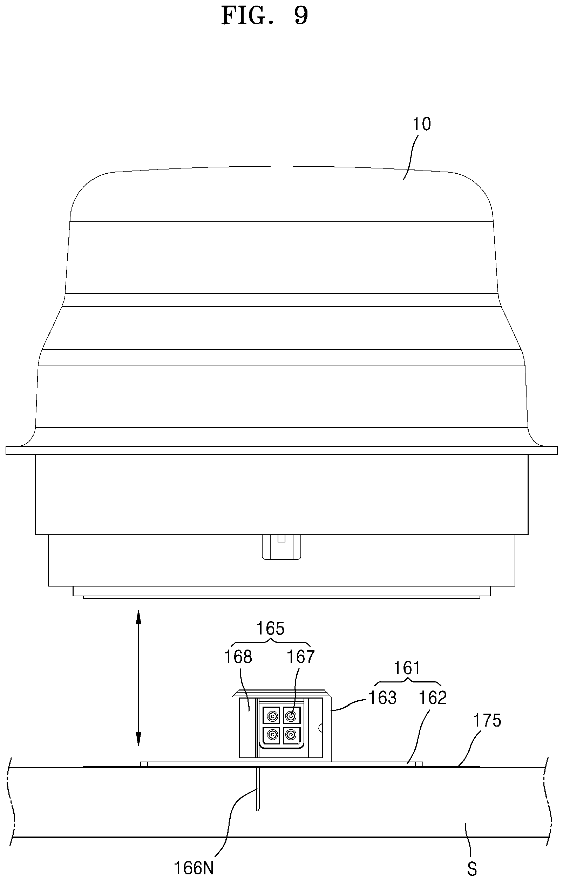

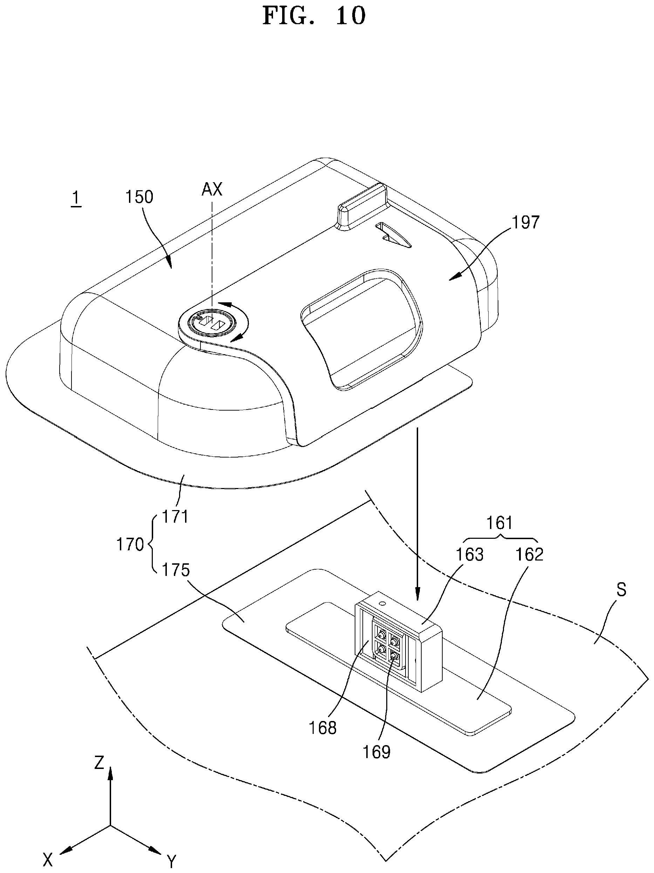

FIG. 1 is a perspective view illustrating a Liquid medicine injection apparatus according to an embodiment of the present disclosure. FIG. 2 is a diagram illustrating a separated state of the Liquid medicine injection apparatus according to an embodiment of the present disclosure. FIG. 3 is a bottom view illustrating a base portion to which an attachment portion is connected, according to an embodiment of the present disclosure. FIG. 4 is a diagram illustrating portion A of FIG. 3. FIG. 5 is a view illustrating a side view of the base portion according to an embodiment of the present disclosure. FIG. 6 is a bottom perspective view illustrating a Liquid medicine injection apparatus according to an embodiment of the present disclosure, from which a first base is removed. FIG. 7 is an exploded view illustrating a sensor portion according to an embodiment of the present disclosure. FIG. 8 is a cross-sectional view taken along line I-I of FIG. 1. FIG. 9 is a diagram illustrating a sensor portion according to an embodiment of the present disclosure. FIG. 10 is a diagram illustrating a path through which a cover portion covers the sensor portion according to an embodiment of the present disclosure.

Referring to FIGS. 1 to 10, a Liquid medicine injection apparatus 1 according to an embodiment of the present disclosure is attached to a medical liquid injection subject, and may inject, to a user, a preset, required amount of medical liquid stored therein.

As a selective embodiment, the Liquid medicine injection apparatus 1 may be mounted on the body of a medical liquid injection subject (hereinafter referred to as a "user").

As a selective embodiment, the Liquid medicine injection apparatus 1 may be mounted on an animal to inject a medical liquid.

The Liquid medicine injection apparatus 1 according to an embodiment of the present disclosure may be used for various purposes depending on the type of medical liquid to be injected. For example, the medical liquid may include an insulin-based medical liquid for diabetic patients, a medical liquid for the pancreas, a medical liquid for the heart, and other various types of medical liquids.

As a selective embodiment, the Liquid medicine injection apparatus 1 may be connected to a remote device (not shown) that is connected in a wired manner or wirelessly.

The user may use the Liquid medicine injection apparatus 1 by operating a remote device located outside the Liquid medicine injection apparatus 1 and monitor the usage status of the Liquid medicine injection apparatus 1.

For example, an amount of a medical liquid injected from the Liquid medicine injection apparatus 1, the number of times of injections of the medical liquid, an amount of the medical liquid stored in a reservoir portion 120, the user's bio information, etc. may be monitored, and based on these, the user may operate the Liquid medicine injection apparatus 1.

As a selective embodiment, the remote device is capable of remotely transmitting and receiving signals from and to the Liquid medicine injection apparatus 1, and the Liquid medicine injection apparatus 1 may receive signals from the remote device and transmit an electrical signal to a driving portion 140, which will be described later.

Although not illustrated in the drawing, the Liquid medicine injection apparatus 1 may include a controller (not shown), and the controller may exchange signals with a remote device and transmit an electrical signal to the driving portion 140. The controller may be provided in a circuit portion 193.

The driving portion 140 may be driven by receiving an electrical signal from the controller, and transmit power to the reservoir portion 120 to deliver a fixed amount of medical liquid from the reservoir portion 120 to a needle portion 130.

When the needle portion 130 receives the medical liquid from the reservoir portion 120, the medical liquid may be discharged into the user's body through the inside of the needle body (reference numeral not specified), which is hollow and penetrates the skin S of the user.

In the present specification, a 'remote device' refers to a communication terminal that is able to use an application in a wired or wireless communication environment. Here, the remote device may be a portable terminal of a user.

Examples of the remote device may include a computer (e.g., desktop, laptop, tablet, etc.), a media computing platform (e.g., cable, satellite set-top box, digital video recorder), a handheld computing device (e.g., PDA, email client, etc.), any form of mobile phone, any form of wearable device that can be used by attaching or mounting the wearable device on the user's body, or any form of other types of computing or communication platform, but the present disclosure is not limited thereto.

The Liquid medicine injection apparatus 1 and the remote device may communicate with each other through a communication network. The communication network refers to a communication network that provides a connection path through which a remote device may transmit or receive data after connecting to a service server (not shown). The communication network may include, for example, wired networks such as LANs (Local Area Networks), WANs (Wide Area Networks), MANs (Metropolitan Area Networks), and ISDNs (Integrated Service Digital Networks), or wireless networks such as wireless LANs, CDMA, Bluetooth, and satellite communications, but the scope of the present disclosure is not limited thereto.

Various modifications may be made, and as a selective embodiment, the remote device may be formed as a single device, or may include a plurality of devices capable of communicating with the Liquid medicine injection apparatus 1.

Referring to FIGS. 1 to 10, the Liquid medicine injection apparatus 1 according to an embodiment of the present disclosure may include the base portion 110, the reservoir portion 120, the needle portion 130, the driving portion 140, the cover portion 150, the sensor portion 160, an attachment portion 170, an alarm unit 180, a battery portion 191, a circuit portion 193, and a lever portion 197.

Referring to FIGS. 2, 5, 6, and 8, the base portion 110 according to an embodiment of the present disclosure is a portion where the reservoir portion 120, the needle portion 130, and the driving portion 140, which will be described later, are installed, and may include a first base 111, a second base 113, and a third base 115.

Referring to FIG. 2, the internal components of the Liquid medicine injection apparatus 1 are disposed on the base portion 110 according to an embodiment of the present disclosure, that is, the reservoir portion 120, the needle portion 130, the driving portion 140, the alarm unit 180, the battery portion 191, and the circuit portion 193 may be disposed. The base portion 110 may be connected to the cover portion 150 on one side thereof (the upper side in FIG. 2), and the other side thereof opposite thereto may be connected to the attachment portion 170, specifically, a first attachment member 171.

Referring to FIGS. 2, 5, and 8, the first base 111 according to an embodiment of the present disclosure may be connected to one side of the cover portion 150 (the lower side in FIG. 2), and the circuit portion 193 may be arranged thereon.

The second base 113, which will be described later, may be arranged on the first base 111, and the second base 113 may be arranged under the internal components such as the reservoir portion 120, the needle portion 130, the driving portion 140, the alarm unit 180, and the battery portion 191 (based on FIG. 2).

As a selective embodiment, the first base 111 may be connected to the cover portion 150 along the outer peripheral surface thereof.

Referring to FIGS. 2, 5, 6, and 8, the second base 113 according to an embodiment of the present disclosure is disposed on the first base 111, and the reservoir portion 120, the needle portion 130, the driving portion 140, the alarm unit 180, and the battery portion 191 may be disposed thereon, and the second base 113 may cover one side of the internal components (the lower side in FIG. 2).

The second base 113 according to an embodiment of the present disclosure may have a hole or groove formed in a preset area, and the reservoir portion 120, the needle portion 130, the driving portion 140, and the battery portion 191 may be formed in the hole or groove.

The second base 113 according to an embodiment of the present disclosure may have an outer peripheral surface (reference numeral not specified) extending outward from the first base 111, and the circuit portion 193 to be described later, specifically, a second circuit 195, may be disposed on the outer peripheral surface.

A first circuit 194 may be arranged on the first base 111, and the first circuit 194 may be arranged between the first base 111 and the second base 113. The second circuit 195 may be connected to the first circuit 194, and may be arranged on the outer peripheral surface of the second base 113 extending from the first base 111 toward the cover portion 150, that is, toward the upper side (based on FIG. 6).

Referring to FIGS. 6 and 8, an installation groove 114 with a predetermined depth may be formed in an outer peripheral surface of the second base 113 opposite the inner peripheral surface of the second base 113 where the internal components are installed, and the second circuit 195 connected to the first circuit 194 may be disposed on the installation groove 114 formed in the outer peripheral surface of the second base 113.

Accordingly, when the cover portion 150, which will be described later, covers the base portion 110 and is connected thereto, a contact portion 155 provided on the cover portion 150 may contact the second circuit 195, and the effect of enabling electrical connection between the circuit portion 193 and the sensor portion 160 after the sensor portion 160, which will be described later, is inserted into the insertion groove 153 formed in the cover portion 150 may be provided.

Referring to FIGS. 5, 6, and 8, as the second circuit 195 according to an embodiment of the present disclosure is disposed on the outer peripheral surface of the second base 113 and exposed to the outside, when the sensor portion 160, which is located outside the base portion 110 and is spatially separable from the base portion 110, is vertically inserted into the insertion groove 153 formed in the cover portion 150, the circuit portion 193 and the sensor portion 160 may be electrically connected to each other through the contact portion 155 provided on the cover portion 150.

In addition, the sensor portion 160 is disposed independently of the base portion 110, and when the medical liquid accommodated in the reservoir portion 120 is used up and the medical liquid needs to be charged or replaced, only the base portion 110 may be separated from the skin S of the user without removing the sensor portion 160 attached to the user's body, thereby improving convenience of use.

Referring to FIGS. 6 and 8, the installation groove 114 having a certain depth and in the shape of a groove may be formed in the second base 113 according to an embodiment of the present disclosure, and the second circuit 195 may be inserted thereinto.

However, the present disclosure is not limited thereto, and various modifications may be made within the technical concept where the second circuit 195 is disposed on the outer peripheral surface of the base portion 110 and exposed to the outside, and while the sensor portion 160 that is spatially separated from the base portion 110 is inserted into the insertion groove 153 formed on the cover portion 150, the sensor portion 160 may be directly or indirectly electrically connected to the second circuit 195.

Referring to FIGS. 2 and 6, the third base 115 according to an embodiment of the present disclosure may cover the second base 113, and may fix positions of the needle portion 130, the driving portion 140, and the battery portion 191 disposed on the second base 113.

Since the third base 115 covers the second base 113 and is fixed in position, the internal components such as the needle portion 130, the driving portion 140, and the battery portion 191 may be prevented from leaving their installation positions on the base portion 110 and transfer of power generated by the driving portion 140 to the reservoir portion 120 may be ensured, thereby improving driving precision.

Referring to FIGS. 2 and 6, the reservoir portion 120 according to an embodiment of the present disclosure accommodates a medical liquid delivered to the needle portion 130, and may deliver the medical liquid to the needle portion 130 by receiving power from the driving portion 140.

Although not shown in the drawing, a movable plunger (not shown) may be disposed inside the reservoir portion 120, and as the plunger moves within the reservoir portion 120, the medical liquid accommodated inside the reservoir portion 120 may be discharged to the outside and delivered to the needle portion 130.

The reservoir portion 120 may be connected to the driving portion 140, and a plunger may be moved by receiving power from the driving portion 140, and through movement of the plunger, a fixed amount of medical liquid may be delivered to the needle portion 130.

The reservoir portion 120 and the driving portion 140 may be connected to each other in a gear manner, but are not limited thereto, and various modifications may be made within the technical concept where power generated by the driving portion 140 is transmitted to the reservoir portion 120 and the medical liquid accommodated in the reservoir portion 120 flows to the needle portion 130.

Various modifications may be made, and as a selective embodiment, the reservoir portion 120 may be installed outside the base portion 110 within the technical concept of delivering the medical liquid to the needle portion 130.

Referring to FIGS. 2 and 6, the needle portion 130 according to an embodiment of the present disclosure may include one or more needle bodies (reference numeral not specified) formed to be injected into the user's body, and may be disposed on the base portion 110, specifically, on the second base 113.

The needle body provided in the needle portion 130 may pass through the base portion 110 and the attachment portion 170 and be inserted into the skin S of the user, and may be hollow so that the discharged medical liquid is injected therethrough into the user.

The needle portion 130 may be connected to the reservoir portion 120 through a conduit (not shown), and the reservoir portion 120, which receives power from the driving portion 140, may transfer a fixed amount of medical liquid to the needle portion 130 through the conduit.

Referring to FIGS. 1 and 2, the needle portion 130 according to an embodiment of the present disclosure may be connected to the lever portion 197, and as the lever portion 197 rotates, the needle portion 130 may rotate about a central axis (Z-axis of FIG. 2) together.

As the needle portion 130 rotates by receiving rotational power from the lever portion 197, the needle body may advance toward the skin S of the user and penetrate the skin S of the user. Accordingly, the effect of allowing the medical liquid delivered from the reservoir portion 120 to the needle portion 130 to be injected into the user's body through the needle body may be provided.

Referring to FIG. 3, a needle cover assembly NC may be disposed in the attachment portion 170 to be described later, specifically, in an area of the attachment portion 170 corresponding to the needle portion 130. The needle cover assembly NC may be connected to the first attachment member 171 while covering holes (reference numeral not specified) formed in the base portion 110 and the first attachment member 171 such that the needle body passes therethrough.

The needle cover assembly NC may be formed of an air-permeable material. The needle cover assembly NC may include a material that allows air discharged from the needle body to pass through, but does not allow the medical liquid to pass through.

For example, the needle cover assembly NC may include various materials such as polytetrafluoroethylene (PTFE), expanded PTFE (ePTFE), Tyvek, and Kynar polyvinylidene fluoride (PVDF).

The needle cover assembly NC may be separated from the attachment portion 170 during an operation of injecting a medical liquid, and as the driving portion 140 is driven, the medical liquid in the reservoir portion 120 may be injected into the user through the needle body.

Referring to FIGS. 2, 6, and 8, the driving portion 140 according to an embodiment of the present disclosure is capable of reciprocating movement in a preset direction, and may transfer power to the reservoir portion 120 such that a medical liquid in the reservoir portion 120 is delivered to the needle portion 130.

The driving portion 140 according to an embodiment of the present disclosure may be electrically connected to the circuit portion 193, and may generate power by receiving an electrical signal through the circuit portion 193, and transmit the power to the reservoir portion 120.

Although not shown in the drawing, the driving portion 140 may include a drive shaft, and the drive shaft may reciprocate in a preset direction and transmit power to the reservoir portion 120.

The driving portion 140 according to an embodiment of the present disclosure may be any type of device that has medical liquid-absorption power and medical liquid-discharging power by electricity. For example, all types of pumps may be used, such as mechanical displacement micropumps and electromagnetic motion micropumps.

A mechanical displacement micropump is a pump that uses the movement of a solid or fluid, such as a gear or diaphram, to create a pressure difference to induce the flow of fluid, and examples thereof include a diaphragm displacement pump, a fluid displacement pump, a rotary pump, etc. An electromagnetic kinetic micropump is a pump that uses energy in the form of electricity or magnetism directly to move a fluid, and examples thereof include an electrohydrodynamic pump (EHD), an electroosmotic pump, a magnetohydrodynamic pump, a magneto hydrodynamic pump, an electro wetting pump, etc.

As a selective embodiment, the driving portion 140 may include a driving housing (reference numeral not specified) which forms the exterior of the driving portion 140 and is hollow, and a fluid may be accommodated in the driving housing, and a membrane (not shown) may be disposed inside the driving housing.

The internal space of the driving housing may be divided, with respect to the membrane, into a first space disposed in a direction away from a hole formed in the driving housing and the drive shaft that reciprocates through the hole, and a second space adjacent to the hole, and the membrane may be located between the first space and the second space.

The driving portion 140 may be include a first electrode body and a second electrode body respectively disposed on both sides of the membrane, and the first electrode body and the second electrode body may contact the circuit portion 193, and receive an electrical signal through the circuit portion 193.

When the driving portion 140 according to an embodiment of the present disclosure receives an electrical signal from the circuit portion 193, a fluid may pass through the membrane through an electrochemical reaction, and by alternately applying different signals, the fluid accommodated in the driving housing may flow from the first space to the second space or from the second space to the first space.

The reciprocating movement of the drive shaft provided in the driving portion 140 may depend on the flow of fluid moving between the first space and the second space, and as the drive shaft reciprocates due to a pressure caused by the flow of the fluid, power may be transmitted to the reservoir portion 120.

In addition, as the reservoir portion 120 receives power at a constant force and cycle from the driving portion 140, a fixed amount of medical liquid may be periodically delivered to the needle portion 130, and the medical liquid may be injected into the body of the user through the needle portion 130, specifically, the needle body.

Referring to FIGS. 1 to 4, 8, and 10, the cover portion 150 according to an embodiment of the present disclosure may cover the base portion 110, and specifically cover the first base 111 and coupled to the first base 111.

The cover portion 150 according to an embodiment of the present disclosure is open on one side thereof (the lower side in FIG. 2) and is hollow, and as the cover portion 150 covers the base portion 110, specifically, the first base 111, the circuit portion 193, the second base 113, the third base 115, the reservoir portion 120, the needle portion 130, the driving portion 140, the alarm unit 180, and the battery portion 191 disposed on the first base 111 may be covered together.

That is, while the cover portion 150 covers the first base 111, the circuit portion 193, the second base 113, the third base 115, the reservoir portion 120, the needle portion 130, the driving portion 140, the alarm unit 180, and the battery portion 191 described above may be located in the internal space of the cover portion 150.

Referring to FIG. 8, the cover portion 150 according to an embodiment of the present disclosure may cover the sensor portion 160, which is spatially separated from the base portion 110, together with the base portion 110.

Referring to FIGS. 3, 4, and 8, a partition wall portion 151 may be formed in the cover portion 150 according to an embodiment of the present disclosure, and the partition wall portion 151 may divide an inner area of the cover portion 150 into multiple areas.

Referring to FIG. 3, the inner area of the cover portion 150 according to an embodiment of the present disclosure may have a first area A1 formed on one side (on the left side in FIG. 3) with respect to the partition wall portion 151, and a second area A2 formed on the other side opposite to the one side of the partition wall portion 151 where the first area A1 is formed (on the right side in FIG. 3).

Referring to FIG. 3, in the first area A1 of the inner area of the cover portion 150 according to an embodiment of the present disclosure, the base portion 110 may be disposed, and the reservoir portion 120, the needle portion 130, the driving portion 140, the alarm unit 180, the battery portion 191, and the circuit portion 193 installed in the base portion 110 may be disposed.

Referring to FIG. 3, the cover portion 150 according to an embodiment of the present disclosure, specifically the first area A1 of the inner area, may cover the base portion 110 and be connected to the base portion 110, specifically the first base 111 while covering the base portion 110, and area A1, and the outer peripheral surface of the second base 113 may be disposed to face the partition wall portion 151.

Referring to FIGS. 3, 4, and 8, the second area A2 may be formed on the other side opposite to the first area A1 with respect to the partition wall portion 151 formed in the cover portion 150 according to an embodiment of the present disclosure (right side based on FIG. 3), and the insertion groove 153 which has a preset depth and is in a shape of a groove may be formed in the second area A2.

Referring to FIGS. 3 and 4, in the insertion groove 153 formed in the second area A2 of the inner area of the cover portion 150 according to an embodiment of the present disclosure, the sensor portion 160 to be described later may be inserted.

Accordingly, when the cover portion 150 covers the base portion 110 and is connected to the base portion 110, the first area A1 covers the base portion 110 with respect to the partition wall portion 151 provided in an inner portion of the cover portion 150, and the internal components such as the reservoir portion 120, the needle portion 130, the driving portion 140, the alarm unit 180, the circuit portion 193, the battery portion 191 arranged on the base portion 110 may be located in the first area A1, and the sensor portion 160 may be arranged in the second area A2 which is spatially separated from the first area A1.

Referring to FIGS. 8 and 10, the sensor portion 160 according to an embodiment of the present disclosure, specifically a frame body 163 and a sensor module 165 within the frame body 163, may be inserted into the insertion groove 153 formed in the second area A2, and then may be arranged to face the partition wall portion 151.

Accordingly, the sensor portion 160 may exist independently and spatially separated from the base portion 110 in which the internal components described above are installed, and may be first located on the skin S of the user as a separate component from the base portion 110 on which the needle portion 130 is installed.

Moreover, using the cover portion 150 connected to the base portion 110, the sensor portion 160 located on the skin S of the user may be covered in a vertical direction (Z-axis direction based on FIG. 10).

In the case of the Liquid medicine injection apparatus 1, as a medical liquid is injected into the user's body, the medical liquid needs to be replenished or the reservoir portion 120 and the battery portion 191 or the like are to be replaced after a certain period of time, and since the replacement cycle of the sensor portion 160 for measurement of a blood sugar level in the body of a user is relatively long, the replacement thereof needs to be separated from replacement of the reservoir portion 120, etc.

The sensor portion 160 according to an embodiment of the present disclosure is not installed on the base portion 110, but is distinguished from the base portion 110 and may be positioned as a separate component, and when replacement of the internal components installed on the base portion 110, such as the reservoir portion 120, is necessary, without changing the sensor portion 160, the base portion 110 and the cover portion 150 covering the base portion 110 may be separated from the skin S of the user.

In addition, the sensor portion 160 includes a sensor needle 166N that penetrates the skin S of the user, and since the position at which the sensor portion 160 is first installed on the skin S of the user may be maintained, the variability that can occur in blood sugar measurement may be minimized, and measurement accuracy and consistency may be improved.

Referring to FIGS. 3, 4, and 8, the partition wall portion 151 according to an embodiment of the present disclosure may be arranged to face the outer peripheral surface of the second base 113 when the cover portion 150 covers the base portion 110 in a vertical direction (Z-axis direction based on FIG. 10).

Referring to FIGS. 5 and 6, on the outer peripheral surface of the second base 113 according to an embodiment of the present disclosure, the circuit portion 193, specifically, the second circuit 195 which is connected to the first circuit 194 disposed on the first base 111 may be disposed, and the partition wall portion 151 may be disposed to face the second circuit 195 during a process in which the cover portion 150 is connected to the base portion 110.

Referring to FIGS. 4 and 8, a through-hole portion 151H may be formed in the partition wall portion 151 according to an embodiment of the present disclosure, and through the through-hole portion 151H, the first area A1 and the second area A2, which are internal spaces formed on both sides of the partition wall portion 151, may be in communication with each other.

Referring to FIGS. 4, 8, and 3, the contact portion 155 may be disposed in the insertion groove 153 formed in the second area A2 of the inner area of the cover portion 150 according to an embodiment of the present disclosure. The contact portion 155 may include a contact body 156 and a contact plate 157.

Referring to FIGS. 3, 4, and 8, the contact plate 157 is disposed within the insertion groove 153 and may contact the sensor portion 160, which will be described later.

The contact plate 157 according to an embodiment of the present disclosure includes a plurality of surfaces 157A, 157B and may be disposed on both sides of the sensor portion 160, and the plurality of surfaces 157A, 157B may be each in contact with the sensor portion 160.

A curved portion 158 may be formed on the contact plate 157 disposed on both sides of the sensor portion 160, and the curved portion 158 may connect the plurality of surfaces 157A and 157B located on both sides of the sensor portion 160 disposed in the insertion groove 153.

Various modifications may be made, and as a selective embodiment, an area connecting the plurality of surfaces 157A and 157B may be formed in a flat shape rather than a curved shape.

Referring to FIGS. 3 and 8, the contact portion 155, specifically the contact plate 157, is in contact with both sides of the sensor portion 160, thereby improving the contact force with respect to the sensor portion 160 and improving transmission capability when transmitting information about blood sugar or the like to the circuit portion 193 as an electrical signal through the contact portion 155.

In addition, as the contact portion 155, specifically the contact plate 157, is in contact with both sides of the sensor portion 160, when replacing the reservoir portion 120 and the base portion 110 on which the internal components such as the reservoir portion 120 and the needle portion 130 are disposed, due to the storage period of the medical liquid accommodated in the reservoir portion 120, the base portion 110 and the cover portion 150 covering the base portion 110 may be coupled to the sensor portion 160 by rotating the base portion 110 and the cover portion 150 by 180 degrees such that a position at which the needle body provided in the needle portion 130 penetrates is changed, the sensor portion 160 being in a state of being installed on the skin S of the user.

Referring to FIG. 8, the base portion 110 and the internal components such as the needle portion 130, the reservoir portion 120, and the driving portion 140 disposed in the base portion 110 are disposed on the left side of the sensor portion 160, and the position at which the needle body provided in the needle portion 130 is inserted into the skin of the user is located on the left side (based on FIG. 8) with respect to the sensor portion 160. However, while the sensor portion 160 is attached to the skin of the user, the base portion 110, the first attachment member 171, and the cover portion 150 coupled to the base portion 110 may be separated from the skin of the user, and the base portion 110 in which the medical liquid is replaced, etc., the first attachment member 171, the cover portion 150, and the internal components may be rotated 180 degrees to cover the sensor portion 160 to be attached to the skin of the user.

Accordingly, the needle body provided in the needle portion 130 may penetrate into a position other than the position where the needle body first penetrated the skin of the user, and based on FIG. 8, the base portion 110 and the needle body provided in the needle portion 130 may be located on the right side of the sensor portion 160.

Referring to FIG. 8, as the contact portion 155 provided on the cover portion 150, specifically the contact plate 157 connected to the contact body 156, may be in contact with and be electrically connected on both sides of the sensor portion 160, the sensor portion 160 may be inserted into the insertion groove 153 to cover the sensor portion 160 by rotating, by 180 degrees, the position of the cover portion 150 in which the insertion groove 153 is formed.

Referring to FIGS. 3, 4, and 8, the contact body 156 according to an embodiment of the present disclosure is connected to the contact plate 157, and may be arranged within the through-hole portion 151H formed in the partition wall portion 151.

Accordingly, the sensor portion 160 and the circuit portion 193, specifically the second circuit 195, may be electrically connected to each other, and blood sugar information of the body of the user, etc. may be transmitted from the sensor portion 160 to the circuit portion 193 as an electrical signal.

Referring to FIGS. 3, 4, and 8, the contact body 156 according to an embodiment of the present disclosure may be disposed in an inner area of the partition wall portion 151, specifically in the through-hole portion 151H, and as the contact body 156 is connected to the contact plate, the position of the contact plate 157 disposed inside the insertion groove 153 may be fixed.

Referring to FIG. 8, the contact body 156 according to an embodiment of the present disclosure may be positioned and fixed inside the through-hole portion 151H formed in the partition wall portion 151, and may be in contact with the inner area of the contact body, specifically, the second circuit 195 located on the second base 113 arranged in the first area A1, when the cover portion 150 is connected to and covers the base portion 110.

As the contact body 156 is in contact with the second circuit 195, the second circuit 195 and the sensor portion 160 which is inserted into the insertion groove 153 formed in the cover portion 150 and then comes into contact with the contact plate 157 connected to the contact body 156 may be maintained in a state in which they are electrically connectable.

The contact portion 155 according to an embodiment of the present disclosure may include a conductive material, and thus, as power is supplied from the battery portion 191, the contact portion 155 may be electrically connected to the circuit portion 193, specifically the second circuit 195, and accordingly the contact portion 155 may also be electrically connected to the sensor portion 160 inserted into the insertion groove 153 formed in the cover portion 150.

Referring to FIGS. 2, 7, 8, 9, and 10, the sensor portion 160 according to an embodiment of the present disclosure is capable of measuring blood sugar, and may include a sensor frame 161 and a sensor module 165.

Referring to FIGS. 2 and 7 to 10, the sensor frame 161 according to an embodiment of the present disclosure forms the exterior of the sensor portion 160 and may include a frame base 162 and a frame body 163.

The sensor frame 161 according to an embodiment of the present disclosure forms the exterior of the sensor portion 160, and the frame base 162 may be connected to the attachment portion 170 to be described later, specifically a second attachment member 175.

The frame body 163 is where the sensor module 165 installed, and may be inserted into the insertion groove 153 formed in the cover portion 150.

As the frame body 163 is inserted into the insertion groove 153, the sensor module 165 disposed in the frame body 163 may contact the contact portion 155, specifically the contact plate 157, and the sensor module 165 may be electrically connected to the circuit portion 193 that may be in contact with the contact portion 155, specifically the second circuit 195.

Referring to FIGS. 2 and 7 to 10, the sensor module 165 according to an embodiment of the present disclosure is installed in the sensor frame 161, and specifically is installed in the frame body 163, and may be located inside the insertion groove 153 formed in the cover portion 150.

Referring to FIGS. 2, 7, and 8, the sensor module 165 may include a sensor body 166, a pin portion 167, and a sensor cover 168.

The sensor body 166 includes the sensor needles 166N, and the sensor needle 166N may penetrate the frame base 162 and the frame body 163 and into the skin S of the user. The sensor needle 166N may penetrate into the user's body and obtain information about blood sugar level in the user's body, and the sensor body 166 may convert the information into an electrical signal and transfer the same to the circuit portion 193 arranged in the base portion 110 through the contact portion 155.

Accordingly, the sensor portion 160 and the base portion 110 are spatially separated from each other, and thus, information about the blood sugar level in the user's body measured by the sensor portion 160 may be transmitted to the circuit portion 193, specifically a controller (not shown).

In the present disclosure, the controller provided in the circuit portion 193 processes the electrical signal received from the sensor portion 160, and detailed descriptions thereof will be omitted.

Referring to FIGS. 2, 7, 8, and 9, the pin portion 167 according to an embodiment of the present disclosure may be in contact with the sensor body 166, and may be provided in plurality.

The pin portion 167 may contact the contact portion 155, specifically the contact plate 157, and may be electrically connected to the contact portion 155, as power is supplied from the battery portion 191.

Referring to FIG. 8, the pin portion 167 according to an embodiment of the present disclosure may contact the sensor body 166 on one side thereof and contact the contact portion 155 on the other side thereof.

As a selective embodiment, the pin portion 167 may be formed in a spring pin manner, and may be compressed while the sensor portion 160 is inserted into the insertion groove 153 formed in the cover portion 150, and as the pin portion 167 is compressed, the sensor body 166 and the contact portion 155, specifically the contact plate 157, may be brought into electrical contact with each other.

Referring to FIG. 7, the pin portion 167 according to an embodiment of the present disclosure may be provided in plurality, and the plurality of pin portions 167 may be disposed on both sides of the sensor body 166, respectively.

Thus, the pin portions 167 may contact a plurality of surfaces provided on the contact plate 157 disposed on both sides of the sensor body 166, and may be compressed while the sensor portion 160 is inserted into the insertion groove 153 formed in the cover portion 150, and the sensor body 166 and the contact plate 157 may be brought into electrical contact with each other.

Referring to FIGS. 2, 7, and 8, the sensor cover 168 according to an embodiment of the present disclosure is installed on the sensor frame 161, specifically the frame body 163, and may cover the pin portion 167 and be connected to the sensor body 166.

The sensor cover 168 may be provided in plurality and may be arranged facing each other on both sides of the sensor body 166. The sensor cover 168 may include a rubber material and may fix the position of the sensor module 165 installed in the frame body 163.

Referring to FIGS. 1, 2, 8, and 10, the attachment portion 170 according to an embodiment of the present disclosure is connected to the base portion 110 and the sensor portion 160, and may be formed to be attachable to the skin S of the user.

Referring to FIGS. 1, 2, 8, and 10, the attachment portion 170 according to an embodiment of the present disclosure may be provided in plurality, and each attachment portion 170 may include a first attachment member 171 and a second attachment member 175.

An adhesive material may be applied to at least one surface of the attachment portion 170, and when the attachment portion 170 is arranged on the skin S of the user, the position of the Liquid medicine injection apparatus 1 installed on the skin S of the user may be fixed using the adhesive material.

Referring to FIG. 2, the first attachment member 171 and the second attachment member 175 according to an embodiment of the present disclosure may be formed as separate components and may be spatially separated from each other.

The first attachment member 171 may be attached to the base portion 110, specifically, to one surface of the first base 111, and the second attachment member 175 may be attached to the sensor portion 160, specifically, to one surface of the frame base 162 provided in the sensor frame 161.

Referring to FIGS. 9 and 10, as the first attachment member 171 and the second attachment member 175 are attached to the base portion 110 and the sensor portion 160, respectively, the sensor portion 160 may be arranged such that the sensor needle 166N penetrates the skin S of the user through a sensor inserter 10.

Next, the sensor portion 160 is covered in a vertical direction such that the sensor portion 160 is inserted into the insertion groove 153 formed in the cover portion 150, and the base portion 110 where the internal components such as the reservoir portion 120, the needle portion 130, the driving portion 140, the alarm unit 180, the battery portion 191, and the circuit portion 193 are arranged, may be arranged on the skin S of the user.

Moreover, as the first attachment member 171 and the second attachment member 175 are spatially separated from each other and attached to the base portion 110 and the sensor portion 160, respectively, if the medical liquid accommodated in the reservoir portion 120 is used up and thus the reservoir portion 120 needs to be replaced or the needle portion 130 or the battery portion 191 is to be replaced, the first attachment member 171, the base portion 110, and the cover portion 150 may be independently separated from the sensor portion 160 and removed from the skin S of the user, which has the effect of allowing the sensor portion 160 to remain installed on the skin S of the user.

Referring to FIG. 2, the alarm unit 180 according to an embodiment of the present disclosure is electrically connected to the circuit portion 193 and may generate sound by receiving an electrical signal from the circuit portion 193.

The controller (not shown) disposed in the circuit portion 193 may receive an electrical signal from the sensor portion 160, and send an electrical signal to the alarm unit 180 when necessary, such as in an emergency situation, depending on the operating state of the Liquid medicine injection apparatus 1, and as sound is generated from the alarm unit 180, the user may recognize a particular state.

Referring to FIGS. 5, 6, and 8, the circuit portion 193 according to an embodiment of the present disclosure is installed on the base portion 110 and may include the first circuit 194 and the second circuit 195.

The circuit portion 193 according to an embodiment of the present disclosure may include a conducting wire so as to be electrically connected to the driving portion 140, the battery portion 191, the alarm unit 180, and the sensor portion 160, and may be formed as, for example, as a printed circuit board (PCB).

The circuit portion 193 described in the present disclosure is not limited to simply conducting wires formed on a substrate, and may include a controller to transmit electrical signals.

The first circuit 194 according to an embodiment of the present disclosure may be installed on the first base 111 and between the first base 111 and the second base 113. The first circuit 194 is disposed on one side (lower side in FIG. 2) of the driving portion 140, the battery portion 191, and the alarm unit 180, and may be electrically connected to the internal components such as the driving portion 140, the battery portion 191, the alarm unit 180, etc.

Referring to FIGS. 5, 6, and 7, the second circuit 195 according to an embodiment of the present disclosure is connected to the first circuit 194 and may be disposed on the outer peripheral surface of the second base 113 disposed on the first base 111.

Referring to FIG. 8, when the cover portion 150 covers and is connected to the base portion 110, the second circuit 195 may be disposed on the base portion 110 facing the partition wall portion 151 formed inside the cover portion 150, specifically on one surface of the second base 113.

That is, the second circuit 195 may be disposed to face the through-hole portion 151H formed in the partition wall portion 151, and may be in contact with the contact portion 155 disposed through the through-hole portion 151H.

Since the second circuit 195 may be in contact with the contact portion 155, the second circuit 195 and the sensor portion 160 may be electrically connected to each other, and information relating to the blood sugar level in the user's body, etc. and obtained by the sensor portion 160 may be transmitted to the circuit portion 193 as an electrical signal.

Referring to FIGS. 8 and 10, the second circuit 195 according to an embodiment of the present disclosure is disposed to face the partition wall portion 151 that partitions the inner area of the cover portion 150, and is able to contact the contact body 156 disposed within the through-hole portion 151H formed in the partition wall portion 151, and thus, when the sensor portion 160 that is installed first on the skin S of the user is inserted into the insertion groove 153 formed in the cover portion 150 installed later, the sensor portion 160 may be electrically connected to the circuit portion 193 through the contact portion 155.

In the present disclosure, the second circuit 195 is formed separately from the first circuit 194 and is electrically connected to the first circuit 194, but is not limited thereto and various modifications may be made, for example, the second circuit 195 may also be formed as a single unit with the first circuit 194 within the technical scope where the second circuit 195 faces the partition wall portion 151 formed in the cover portion 150 covering the base portion 110, and is electrically connected to the sensor portion 160 as the sensor portion 160 is vertically inserted into the insertion groove 153 formed in the cover portion 150.

Referring to FIGS. 2 and 6, the battery portion 191 according to an embodiment of the present disclosure may supply electricity to the Liquid medicine injection apparatus 1 to activate each component. Although the drawing shows a single battery portion 191, the present disclosure is not limited thereto and the battery portion 191 may be set in various manners according to the capacity, usage range, usage time, etc. of the Liquid medicine injection apparatus 1.

The battery portion 191 according to an embodiment of the present disclosure may be disposed adjacent to the driving portion 140 and may supply electricity to the driving portion 140. Additionally, the battery portion 191 may be connected to the circuit portion 193 and supply power to the sensor portion 160.

The controller (not shown) installed on the circuit portion 193 may receive power from the battery portion 191 and receive electrical signals from the sensor portion 160 electrically connected to the circuit portion 193.

The controller may measure data on the driving performance of the driving portion 140, the amount of medical liquid stored in the reservoir portion 120, and the amount of medical liquid injected into the user, based on electrical signals measured by the sensor portion 160.

Referring to FIGS. 1, 2, and 10, the lever portion 197 according to an embodiment of the present disclosure is disposed on the cover portion 150 and may be rotatably connected to the cover portion 150.

The lever portion 197 may be connected to the needle portion 130, and as the user rotates the lever portion 197 based on a central axis of rotation AX, the needle portion 130 connected to the lever portion 197 may be rotated in conjunction with the lever portion 197, and the needle body provided in the needle portion 130 may penetrate the skin S of the user.

The operating principle and effects of the Liquid medicine injection apparatus 1 according to an embodiment of the present disclosure as described above will now be described.

Referring to FIGS. 1 to 10, the Liquid medicine injection apparatus 1 according to an embodiment of the present disclosure may include the base portion 110, the reservoir portion 120, the needle portion 130, the driving portion 140, the cover portion 150, the sensor portion 160, the attachment portion 170, the alarm unit 180, the battery portion 191, the circuit portion 193, and the lever portion 197.

In the Liquid medicine injection apparatus 1 according to an embodiment of the present disclosure, the sensor portion 160 is not installed on the base portion 110, but is provided separately from the base portion 110 and may be first installed on the skin S of the user.

Referring to FIG. 9, the user may hold the sensor inserter 10 on which the sensor portion 160 is installed and install the sensor portion 160 at a position where the Liquid medicine injection apparatus 1 is to be installed.

Although not shown in the drawing, the sensor inserter 10 on which the sensor portion 160 is installed may be brought into contact with the skin S of the user and then the sensor portion 160 may be moved toward the skin S of the user through a push operation in a direction toward the skin S of the user.

Referring to FIG. 9, through the above operation, the sensor needle 166N provided in the sensor portion 160 may penetrate the skin S of the user, and the sensor portion 160 may be arranged on the skin S of the user.

The second attachment member 175 may be connected to the sensor portion 160, specifically, one surface (lower surface of FIG. 10) of the frame base 162 provided in the sensor frame 161, and as the second attachment member 175 to which an adhesive material is applied is adhered to the skin S of the user, the sensor portion 160 may be installed on the skin S of the user while the sensor needle 166N is maintained in a state of penetrating the skin S.

Referring to FIG. 10, the sensor portion 160 according to an embodiment of the present disclosure is first installed on the skin S of the user, and the user may cover, by using the cover portion 150 coupled to the base portion 110 in a vertical direction (Z-axis direction based on FIG. 10), the sensor portion 160 coupled to the base portion 110 so that the sensor portion 160 is inserted into the insertion groove 153 formed in the cover portion 150.

Referring to FIG. 10, the first attachment member 171 may be connected to one surface (lower surface of FIG. 10) of the base portion 110 according to an embodiment of the present disclosure, and as with the second attachment member 175, an adhesive material may be applied to the first attachment member 171, and thus, the base portion 110 and the cover portion 150 to which the base portion 110 is coupled may be fixed in position at a position at which the sensor portion 160 is covered.

Referring to FIGS. 3, 4, and 8, the partition wall portion 151 may be provided in the cover portion 150 according to an embodiment of the present disclosure, and the inner area of the cover portion 150 may be divided into the first area A1 and the second area A2 due to the partition wall portion 151.

Referring to FIG. 3, the base portion 110 may be connected to the first area A1, and the internal components such as the reservoir portion 120, the needle portion 130, the driving portion 140, the attachment portion 170, the alarm unit 180, the circuit portion 193, and the battery portion 191 may be disposed in the first area A1.

Referring to FIGS. 3 and 8, the insertion groove 153 may be formed in the second area A2 opposite the first area A1 with respect to the partition wall portion 151, and the sensor portion 160 may be inserted into and arranged in the insertion groove 153.

Referring to FIG. 10, the sensor portion 160 may be pre-installed on the skin S of the user, and the sensor portion 160 may be vertically inserted into the insertion groove 153 formed in the cover portion 150.

Referring to FIGS. 3, 4, and 8, the cover portion 150 according to an embodiment of the present disclosure may include the contact portion 155, the contact portion 155 may include the contact body 156 and the contact plate 157, the contact body 156 may be disposed within the through-hole portion 151H formed in the partition wall portion 151, and the contact plate 157 may be connected to the contact body 156 and inserted into the contact body 156.

Referring to FIG. 8, the sensor portion 160 may be inserted into the insertion groove 153 formed in the cover portion 150 and may be contact the contact plate 157, and as the contact body 156 that is connected to the contact plate 157 and passes through the partition wall portion 151 to be arranged is in contact with the circuit portion 193, specifically, the second circuit 195, the sensor portion 160 and the second circuit 195 may be electrically connected to each other.

Referring to FIGS. 1 and 10, the sensor portion 160 is inserted into the insertion groove 153 formed in the cover portion 150, and the Liquid medicine injection apparatus 1 is attached to the skin S of the user, and then by rotating the lever portion 197, the user may allow the needle body provided in the needle portion 130 to penetrate the skin S of the user.

Referring to FIG. 8, the sensor needle 166N provided in the sensor portion 160, which is spatially separated from the base portion 110, may penetrate the skin S of the user and measure blood sugar in the user's body.

Power may be supplied from the battery portion 191 installed in the base portion 110, and an electrical signal may be transmitted to the driving portion 140 through the circuit portion 193. Power may be generated as an electrical signal is transmitted to the driving portion 140, and as the power is transmitted to the reservoir portion 120, and a fixed amount of medical liquid may flow from the reservoir portion 120 to the needle portion 130.

As a selective embodiment, as an electrical signal is applied to the driving portion 140, an electric and chemical reaction occurs in the fluid accommodated in the driving portion 140, and power may be generated due to a pressure generated by the flow of the fluid between the first space and the second space formed on both sides of the membrane disposed inside the driving portion 140.

As the power generated from the driving portion 140 according to an embodiment of the present disclosure is transmitted to the reservoir portion 120, a fixed amount of medical liquid may be transferred from the reservoir portion 120 to the needle portion 130 and injected into the body of the user through the needle body.

In the Liquid medicine injection apparatus 1 according to an embodiment of the present disclosure, the sensor portion 160 may be spatially separated from the base portion 110 and provided independently thereof, and as the sensor portion 160 is inserted into the insertion groove 153 formed in the cover portion 150, the circuit portion 193 and the sensor portion 160 may be electrically connected to each other.

In addition, because the sensor portion 160 is spatially separated from the base portion 110, the sensor portion 160 may be installed separately on the skin S of the user first, and then the reservoir portion 120 and then the base portion 110, on which the internal components such as the reservoir portion 120, the needle portion 130, the driving portion 140 may be installed on the skin S of the user.

In addition, when it is necessary to replenish the medical liquid or replace the battery portion 191 and the needle portion 130, the base portion 110 may be independently removed from the skin S of the user to be replaced while the sensor portion 160 is maintained installed on the skin S of the user, which has the effect of improving convenience of use.

The scope of the present disclosure is defined not by the embodiments described above, but by the claims and their equivalents, and all variations within the scope of the claims and their equivalents are to be construed as being included in the present disclosure.

### Industrial Applicability

According to the present disclosure, a Liquid medicine injection apparatus is provided. Additionally, the embodiments of the present disclosure may be applied to industrial devices for injecting a medical liquid into a patient's body.

## Claims

1. A Liquid medicine injection apparatus comprising:
a needle portion having one or more needle bodies configured to be injected into a user's body;
a reservoir portion configured to accommodate a medical liquid and receive power from a driving portion to deliver the medical liquid to the needle portion;
a base portion on which the reservoir portion, the needle portion, and the driving portion are installed;
a cover portion covering the base portion; and
a sensor portion configured to measure blood sugar,
wherein, in the cover portion, an insertion groove which has one side open and has a preset depth is formed, and
the sensor portion is insertable into the insertion groove and is spatially separable from the base portion.

2. The Liquid medicine injection apparatus of claim 1, further comprising an attachment portion which is connected to the base portion and the sensor portion and attachable to the user.

3. The Liquid medicine injection apparatus of claim 2, wherein the attachment portion comprises:
a first attachment member connected to one side of the base portion; and
a second attachment member that is formed as a separate component from the first attachment member and is connected to one side of the sensor portion.

4. The Liquid medicine injection apparatus of claim 1, further comprising a lever portion rotatably installed on the cover portion and configured to transmit rotational power to the needle portion.

5. The Liquid medicine injection apparatus of claim 1, wherein the sensor portion comprises:
a sensor frame that is insertable into the insertion groove; and
a sensor module installed in the sensor frame and comprising a sensor needle.

6. The Liquid medicine injection apparatus of claim 5, wherein the sensor module comprises:
a sensor body in which the sensor needle is formed;
a pin portion connected to the sensor body and comprising a conductive material; and
a sensor cover that covers the pin portion and is connected to the sensor frame.

7. The Liquid medicine injection apparatus of claim 6, wherein the pin portion and the sensor cover are each provided in plurality and are disposed on both sides of the sensor body.

8. The Liquid medicine injection apparatus of claim 1, further comprising a circuit portion electrically connected to the driving portion.

9. The Liquid medicine injection apparatus of claim 8, wherein the base portion comprises:
a first base connected to one side of the cover portion and on which the circuit portion is disposed; and
a second base disposed on the first base and configured to fix positions of the reservoir portion, the needle portion, and the driving portion.

10. The Liquid medicine injection apparatus of claim 9, wherein the circuit portion comprises:
a first circuit disposed on the first base; and
a second circuit connected to the first circuit and disposed on an outer peripheral surface of the second base extending from the first base toward the cover portion.
